# EUROPEAN PATENT APPLICATION

(11) **EP 4 686 480 A1**
(43) Date of publication of application: **04.02.2026**
(21) Application number: 24192291.3
(22) Date of filing: 01.08.2024
(51) Int. Cl.: A61K 39/108

(54) **LIVE ATTENUATED E. COLI VACCINE**

(71) Applicant: Institut für Virologie und Immunologie (IVI), 3147 Mittelhäusern (CH); Universität Bern, 3012 Bern (CH)
(72) Inventor: SCHÄREN, Olivier Pascal, 3008 Bern (CH); STÖCKLI, Sabrina, 3008 Bern (CH); RUGGLI, Nicolas, 3147 Mittelhäusern (CH); SUMMERFIELD, Artur, 3147 Mittelhäusern (CH); HAPFELMEIER-BALMER, Siegfried Hektor, 3065 Bolligen (CH)
(74) Representative: HGF

(57) **Abstract**

The present invention relates to live attenuated bacteria which persist in a subject and their use in vaccine compositions. In particular, the invention is directed to mutant strain *E .coli* vaccines which are useful for poultry, and especially chickens.

## Description

### Field of Invention

The present invention relates to live attenuated bacteria which persist in a subject and their use in vaccine compositions. In particular, the invention is directed to mutant strain *E .coli* vaccines which are useful for poultry, and especially chickens.

### Background to the Invention

The idea of interfering with metabolic pathways that are exclusive to the bacterial kingdom and absent from the eukaryotic host to control bacterial growth has proven effective in various established and experimental treatment approaches, such as antibiotics or approved and experimental GMO based vaccines. Our laboratory has been studying bacterial strains with genetic modifications that target bacterial cell wall metabolism and deprive the bacterium of cell wall building blocks essential for survival and their potential to induce a specific immune response. Without exogenous supplementation of the targeted metabolites the bacteria are unable to grow. Such mutants have not been designed, studied nor applied successfully in the context of avian pathogenic *E. coli* (APEC). APEC are a subset of extraintestinal *E. coli* (ExPEC) and a heterogenous group of pathogenic *E. coli* causing a variety of extraintestinal infections in poultry and being a threat to the poultry industry as well as to the entire value chain including the end customer.

Colibacillosis is a common systemic disease of economic importance in poultry and occurs worldwide. *Escherichia coli* (*E. coli*) infection occurs as an acute fatal septicemia or subacute pericarditis and airsacculitis, as well as perihepatitis, arthritis, and also cellulitis. Among bacterial infections, colibacillosis is very often the first cause of morbidity and mortality in poultry. Large numbers of *E. coli* are maintained in the poultry house environment through fecal contamination. Systemic infection occurs when large numbers of pathogenic *E. coli* gain access to the bloodstream via the respiratory tract or intestine. Bacteremia progresses to septicemia and death, or the infection extends to serosal surfaces, pericardium, joints and other organs. Conventionally raised chicken harbour a variety of bacteria.

In terms of probiotic vaccine development, an important development hurdle is that with increasing complexity of the microbiota of a subject colonization resistance increases. Previous unpublished results from our lab have shown that with increasing complexity of the microbiota in mice the stable niche engraftment of metabolically impaired *Salmonella* Typhimurium is not guaranteed. Our vision is to combine both, immunogenicity and niche competition in one live vaccine strain. In order to achieve this, we needed a strain that is still virulent, metabolically deficient and therefore highly dependent on an intact microbiota. At the same time the strain needs to be applicable in a relevant window of opportunity where it still can engraft and subsequently persist in a dedicated niche. This should hold true also with increasing complexity of the microbiota as the vaccinated individuum ages and acquires more bacteria. The developing microbiota thus becomes part of the vaccination process by locally maintaining the vaccine in its biologically effective form near its natural point of attack in the intestine.

As we have shown previously, cell wall auxotrophic *Salmonella* Typhimurium (STmAux) can be an efficient colonizer of mice with a defined, simple microbiota while inducing a robust immune response. With increasing complexity of the microbiota come more complex intermicrobial interactions as well as more extensive consumption of nutrients and extensive occupation of ecological niches. The logical hypothesis for such an impaired organism would be to assume that it has a massively decreased fitness and is not able to persist in a competitive setting found in conventional animals. Hence similarly attenuated bacteria have found their hypothetical application only in settings where they are systemically applied (i.e. injection into the hosts body) and cannot replicate due to the lack of nutrients.

The means by which a warm blooded animal, including a human, overcomes microbial pathogenesis is a complex process. Immunity to microbial pathogenesis is one means by which a warm blooded animal avoids pathogenesis, or suffers a less intense pathogenic state. Incomplete immunity to a given pathogen results in morbidity and mortality in a population exposed to a pathogen. It is generally agreed that vaccines based on live but attenuated micro-organisms (live attenuated vaccines) induce a highly effective type of immune response. Such vaccines have the advantage that, once the animal host has been vaccinated, entry of the microbial pathogen into the host induces an accelerated recall of earlier, cell-mediated or humoral immunity which is able to control the further growth of the organism before the infection can assume clinically significant proportions. Vaccines based on a killed pathogen (killed vaccine) are generally conceded to be unable to achieve this type of response. However, vaccines that contain a live pathogen present, depending on the level of attenuation, the danger that the vaccinated host upon vaccination may contract the disease against which protection is being sought. Therefore, it would be desirable to have a vaccine that possesses the immunising attributes of a live micro-organism but that is not capable of causing undesirable side effects upon vaccination.

It is important to note that the effective use of an attenuated bacterial strain as a vaccine candidate cannot be predicted merely by such level of attenuation. In this regard, the general approach for attenuating bacteria is the removal of one or more virulence factors (genetic modified organisms - GMOs), in most cases, however, virulence factors also play a role in inducing immunity as protective epitopes. In those cases, deletion of virulence factors unavoidably impairs the immunogenic capacities of the bacterium. This is of course an unwanted situation. Therefore, a live vaccine should preferably retain the antigenic complement of the wild type strain.

Moreover, once attenuation level is established, the immune response to a particular type of vaccine candidate and the success of a vaccine composition including such micro-organisms may still be influenced by many factors as detailed below:
a. The live attenuated vaccine strain should preferably have substantially no probability for reverting to its original state (usually a virulent wild type strain) and none of the genes manipulated should be complemented by other genes causing the bacteria to be capable of causing disease (stable mutations are preferred).
b. The presence of endotoxins in a live vaccine can be a disadvantage if not considered as these molecules can cause serious systemic reactions. Also, the administration of whole-cell vaccines is a classical risk factor for local reactogenicity (severe pain, local swelling and edema, panniculitis or ulcer, etc).
c. The viability and fitness of the attenuated GMO should not be drastically affected, as some replication is expected to occur in the body to create enough of the micro-organism and its antigens to stimulate the immune system. In fact, any mutation in a gene may interfere with replication or may damage the live micro-organism in the vial, causing the vaccine to be ineffective.
d. Moreover, gene sharing or protein moonlighting - a phenomenon by which a protein can perform more than one function - should be considered when selecting a gene target for genetic manipulation.
e. In addition, the type of immune response elicited by a vaccine may not be appropriate to provide an adequate protection against infection (vaccine failure). The specific requirements for an effective vaccine will vary according to the nature of the pathogen. In the case of extracellular pathogens, the major antibodies provide adaptative mechanisms for the defense of the organism, while the presence of T cells is essential in controlling intracellular organisms. In consequence, live attenuated vaccines serve as better immunogens that killed bacteria or subunit compositions by means of simple multiplication, as well as by the modifications of bacterial antigens that occur during in vivo infection. Thereby, a live attenuated strain could engender a broader and adequate immune response, especially in the intracellular phase. In this sense, gene-targeted strategies of attenuation should be carefully tested in vaccine candidates, as the ability of the manipulated bacteria to exploit the natural pathways of infection could be potentially impaired and not trigger a broadly protective immune response.
f. In addition, irrespectively of the attenuation level or the type of immune response elicited, the number of doses administrated to achieve an acceptable level of protection with a specific GMO (effective and lasting) can be unsustainable for a vaccine schedule.
g. Furthermore, the route of administration of a vaccine can determine the type of immune response mounted and to be crucial for its success. Consequently, once attenuation level is established for a GMO, the site of vaccine administration could determine the failure or success of vaccination

In summary, a live vaccine should be sufficiently attenuated (or a-virulent) to avoid unacceptable pathological effects, but on the other hand it must elicit an adequate immune response capable of conferring a lasting protection in the host against the disease (protective immunity) independently of the bacterial strain.

Demonstrating that a live vaccine is sufficiently attenuated (or a-virulent) to avoid unacceptable pathological effects and elicits an adequate immune response capable of conferring a lasting protection in the host against the disease (protective immunity) independently of the bacterial strain, is not an easy task.

### Summary of Invention

Here we made the surprising and unexpected discovery of an effective vaccine strain being genetically modified to maximize immunogenicity while impairing its self-sustainability (i.e. no interference with virulence factors). Here the combination of deleted genes being necessary to prevent growth without the corresponding metabolites, while being sufficient to enable efficient supplementation of the missing metabolites by the normal microbiota.

At the same time this combination of gene deletions does not impair the fitness of the strain in association with the host species making it evolutionarily stable and therefore improbable to revert the phenotype. The combination of gene deletions being self-limiting even in the very unlikely case of pathogenicity. Administration of said vaccine at a time point that is suitable for successful niche engraftment through said strain.

Our invention therefore has a strong technology platform character translatable to multiple current, emerging, neglected, and future enteropathogens. The invention's effectivity is not expected to be affected by antibiotic multi-resistance mechanisms and thus offers an alternative, preventive strategy to manage emerging antimicrobial resistance.

In accordance with an aspect of the present invention there is provided an *Escherichia Coli* attenuated by a mutation in any one of the following genes alr, asd, metC, dadX and murl, wherein the mutation renders the corresponding alr, asd, metC, dadX and murl protein nonfunctional.

In another aspect of the invention there is provided an immunogenic composition comprising the *Escherichia Coli* of the present invention.

In another aspect of the invention there is provided a vaccine composition comprising an immunogenically effective amount of at least one *Escherichia Coli* of the present invention and a pharmacologically acceptable carrier.

In another aspect of the invention there is provided a method for attenuating *Escherichia Coli,* the method comprising mutating at least one alr, asd, metC, murl and dadX gene and wherein the attenuated bacteria of the present invention persists in a subject.

In another aspect of the invention there is provided a method of prevention or amelioration of a disease in a subject, the method comprising administering a therapeutically effective dose of a vaccine composition or immunogenic composition according to the present invention to the subject.

In another aspect of the invention there is provided a method of prophylaxis of a disease, the method comprising administering a therapeutically effective dose of a vaccine composition or immunogenic composition according to the present invention to a subject in need of prophylaxis.

In another aspect of the invention there is provided the use of a vaccine composition for the treatment or prophylaxis of disease wherein the vaccine composition comprises at least one *Escherichia Coli* of the present invention.

Throughout the description and claims of this specification, the words "comprise" and "contain" and variations of them mean "including but not limited to", and they are not intended to (and do not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

Various aspects of the invention are described in further detail below.

### Brief Description of the Drawings.

Exemplary arrangements of the disclosure shall now be described with reference to the drawings in which:

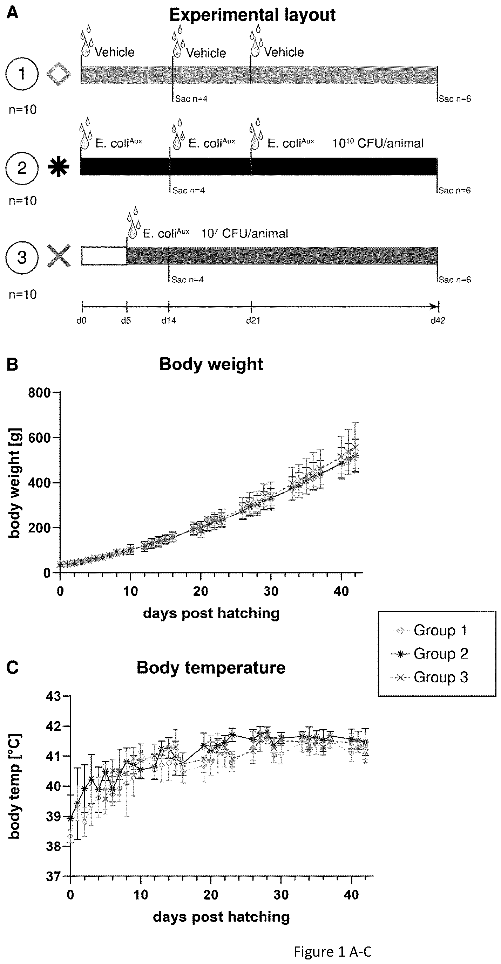

**Figure 1** **Safety of cell wall auxotrophic APEC field isolates:** (A) Experimental layout (n=10 animals per group until d14, n=6 animals per group until day 42). (B) Weight increase of animals over the course of 42 days. (C) Body temperature over the course of 42 days. Statistics: In B and C means are shown and error bars represent standard deviation, n=10 animals per group for data until d14, n=6 animals per group for data until day 42.

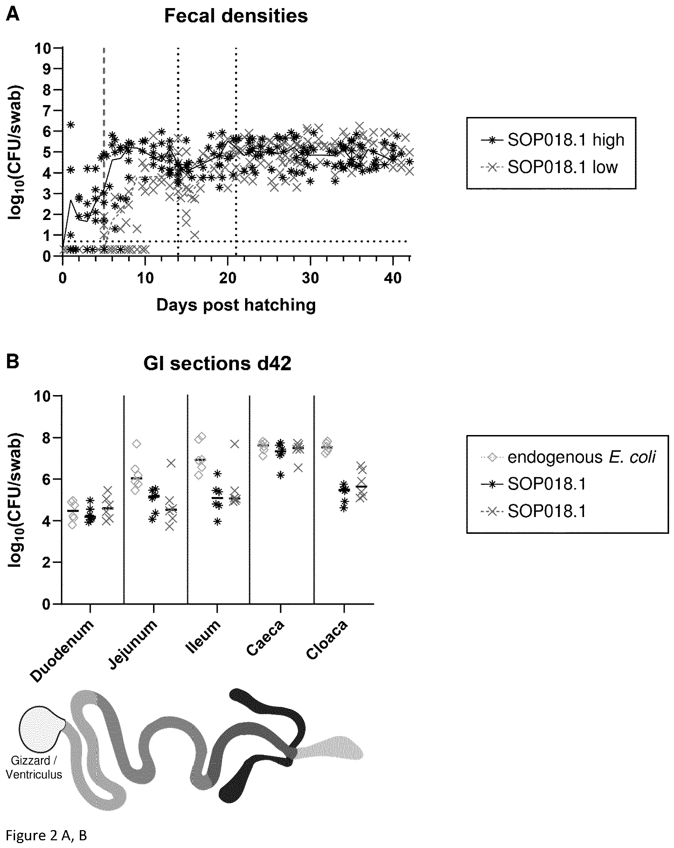

**Figure 2** **Niche engraftment and colonization:** (A) Fecal densities of SOP018.1 in chickens from group 2 (black asterisk) and group 3 (gray cross) respectively (n=10 animals per group until d14, n=6 animals per group until day 42). Vertical dotted lines indicate the day of inoculation for the respective group. (B) Densities of endogenous *E. coli* (group 1) and SOP018.1 (groups 2 and 3) normalized to weight of content in the different sections of the chicken's gastrointestinal tract as illustrated in the schematic below the graph (n=6 animals per group).

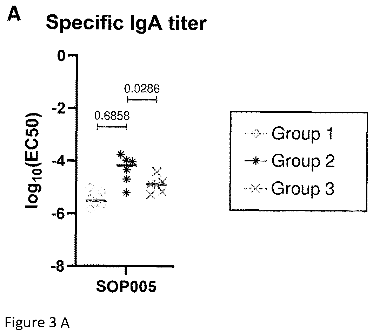

**Figure 3** **Immunogenicity:** (A) IgA titer against the wild type of the vaccine strain SOP018.1 (i.e. SOP005). Statistics: Groups were compared to the control (group 1) using an ordinary one way ANOVA with Dunnett's post hoc test.

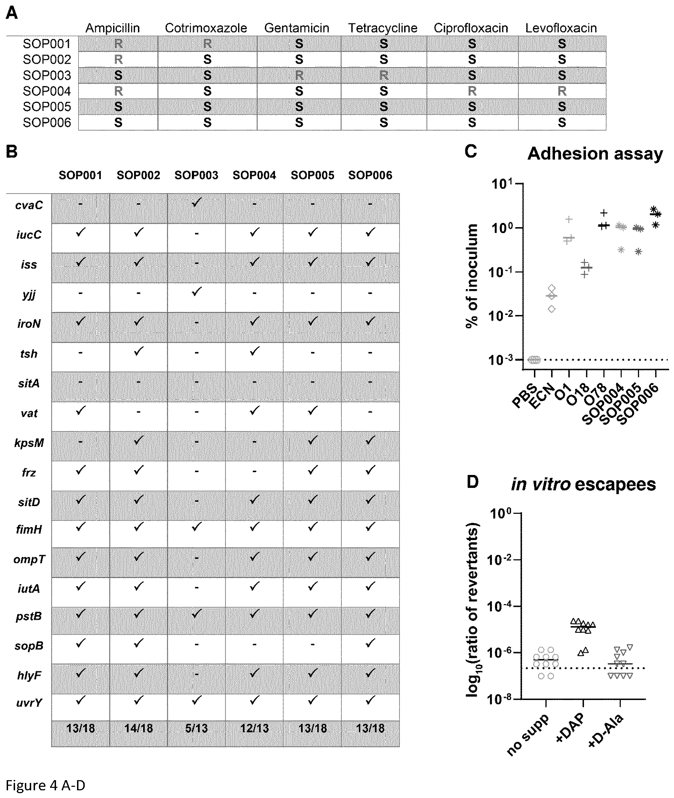

**Figure 4** **Isolation of APEC strains:** (A) Overview of resistances found in field isolates. (B) Overview of virulence factors found in field isolates. (C) Adhesion assay of selected isolates and type strains (DSMZ) on HeLa cells. Each dot represents a biological replicate which is calculated from the mean of three technical replicates. (D) LFL-agar in vitro escapee assay. Each dot represents the ratio of growing revertants under specified conditions towards the number of CFU inoculated under the specified conditions. Statistics: the dotted lines in C and D represent the limit of detection.

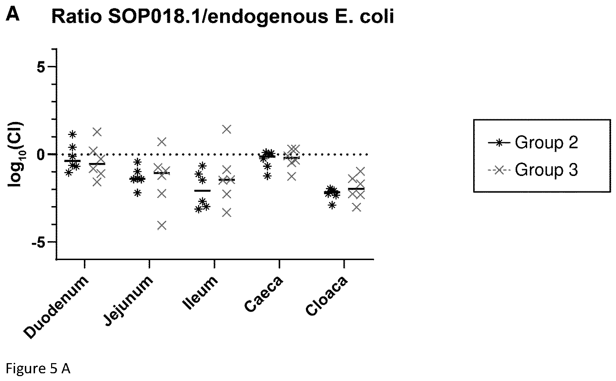

**Figure 5** **Ratio of SOP018.1 vs endogenous *E*. *coli* in the same individual:** (A) Ratio of vaccine strain and endogenous *E. coli* in the different sections of the chicken's gastrointestinal tract (n=6 animals per group).

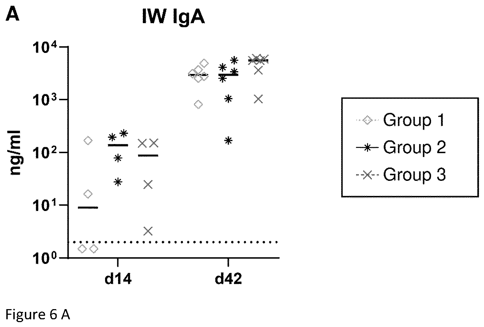

**Figure 6** **Immunogenicity:** (A) IgA levels determined by ELISA on days 14 (n=4 animals per group, ) and 42 (n=6 animals per group) of experiment determined in control animals (group 1, gray diamonds), group 2 (black asterisk) and group 3 (gray cross).

The patent, scientific and technical literature referred to herein establish knowledge that was available to those skilled in the art at the time of filing. The entire disclosures of the issued patents, published and pending patent applications, and other publications that are cited herein are hereby incorporated by reference to the same extent as if each was specifically and individually indicated to be incorporated by reference. In the case of any inconsistencies, the present disclosure will prevail.

Various aspects of the invention are described in further detail below.

### Specific Description

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionary of many of the terms used in the invention. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular terms "a", "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

The principle behind vaccination is to induce an immune response in the recipient, thus providing protection against subsequent challenge with a pathogen. This may be achieved by inoculation with a live attenuated strain of the pathogen, i.e. a strain having reduced virulence such that it does not cause the disease caused by the virulent pathogen while still stimulating a broad immune response.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

In a first aspect of the present invention there is provided an *Escherichia Coli* attenuated by a mutation in any one of the following genes alr, asd, metC, dadX and murl, wherein the mutation renders the corresponding alr, asd, metC, dadX and murl protein nonfunctional.

In the context of the present invention, the term " *Escherichia Coli"* are characterized by being a gram-negative, facultative anaerobic, rod-shaped, coliform bacterium of the genus *Escherichia* that is commonly found in the lower intestine of warm-blooded organisms. A common subdivision system of *E. coli,* but not based on evolutionary relatedness, is by serotype, which is based on major surface antigens (O antigen: part of lipopolysaccharide layer; H: flagellin; K antigen: capsule), e.g. O157:H7). It is, however, common to cite only the serogroup, i.e. the O-antigen. At present, about 190 serogroups are known. The common laboratory strain has a mutation that prevents the formation of an O-antigen and is thus not typeable. *Escherichia coli* of serotypes 036, 078, and 0109, which are highly pathogenic and are suitable for preparing vaccines for treating avian colibacillosis. The most common serotypes include O1, 02, 018 and O78. Another object of the present invention is to provide a vaccine for avian colibacillosis, which can induce the production of higher antibody levels and protect against infection by the currently prevalent virulent strains of *Escherichia coli* O78, O36, 0109 and other serotypes.

In the context of the present invention, the term "vaccine" refers to an antigenic preparation used to establish an immune system response to a disease.

In the context of the present invention, the term "alr" is understood as being synonymous with the term "alanine racemase". In the context of the present invention, mutation in air gene is defined as the absence of locus air in the chromosome of *Escherichia Coli* strain.

In the context of the present invention, the term "asd" is understood as being synonymous with the term " aspartate semialdehyde dehydrogenase". Aspartate β-semialdehyde dehydrogenase (ASADH) is an enzyme involved in the diaminopimelate pathway of lysine biosynthesis. It is essential for the viability of many bacteria, including pathogenic bacteria. In the context of the present invention, mutation in asd gene is defined as the absence of locus asd in the chromosome of *Escherichia Coli* strain.

In the context of the present invention, the term "metC" is understood as being synonymous with the term " Cystathionine β-lyase". Cystathionine β-lyase catalyzes the breakdown of cystathionine to homocysteine, the penultimate step in methionine biosynthesis. In the context of the present invention, mutation in metC gene is defined as the absence of locus metC in the chromosome of *Escherichia Coli* strain.

In the context of the present invention, the term "dadX" is understood as being synonymous with the term "alanine racemase". *E. coli* has two alanine racemases, DadX and Alr. DadX is responsible for most of the alanine racemase activity in the cell and is inducible by either form of alanine, and repressible by glucose. Alr, in contrast, is constitutively expressed. In the context of the present invention, mutation in DadX gene is defined as the absence of locus DadX in the chromosome of *Escherichia Coli* strain.

In the context of the present invention, the term "murl" is understood as being synonymous with the term " glutamate racemase". The bacterial cell wall is a highly cross-linked polymeric structure consisting of repeating peptidoglycan units, each of which contains a novel pentapeptide substitution which is cross-linked through transpeptidation. The incorporation of d-glutamate as the second residue is strictly conserved across the bacterial kingdom. Glutamate racemase, a member of the cofactor-independent, two-thiol-based family of amino acid racemases, has been implicated in the production and maintenance of sufficient d-glutamate pool levels required for growth. In the context of the present invention, mutation in murl gene is defined as the absence of locus murl in the chromosome of *Escherichia Coli* strain.

The mutations utilised to create a non-functional protein may be an insertion, deletion or substitution mutations or a combination thereof, provided that the mutation leads to the failure to express a functional protein. The proteins may have multiple functions, a non-functional protein includes a protein that is defective in at least one of its functions.

In a preferred embodiment the mutation is a knockout mutation where at least a substantial portion of the nucleic acid encoding the protein is deleted. An insertion mutation may be made for example by homologous recombination, transposon mutagenesis or sequence tag mutagenesis. Live attenuated bacteria for use according to the invention may be obtained in a number of ways. For example live attenuated bacteria may be prepared by treatment of wild-type bacteria with mutagenic agents such as purine or pyrimidine analogues, ultraviolet light, ionizing radiation, DNA intercalating agents, temperature treatment, transposon mutagenesis. These methods do not target mutations to specific genes and thus necessitate screening of the treated bacteria for attenuation by methods known in the art such as by DNA sequence analysis of target genes in combination with pathogenicity studies.

Recombinant DNA technology using known methods such as site directed mutagenesis may be used to introduce a mutation at a predetermined site of a specific gene. Site directed mutagenesis maybe used to introduce a mutation such as an insertion, a deletion, a replacement of one or more nucleotides such that the mutated gene no longer expresses a functional protein. Such mutations may for instance be made by deletion of a number of nucleic acids.

Deletions of as few as a single base, thus creating a frame shift, can render a protein non-functional. In some embodiments larger numbers of bases are deleted. In other embodiments the majority or all of a gene encoding the protein may be deleted. Mutations that introduce a stop-codon or frame- shift are suitable for obtaining a non-functional protein in the present invention.

Genes encoding proteins comprise not only the coding sequence, but include regulatory sequences for example promoters. Genes also include regions essential for correct mRNA translation for example ribosome binding sites. Accordingly, the live attenuated bacteria may contain mutations not only in the coding regions but also or alternatively in sequences essential for transcription and translation such as promoters and ribosome binding sites. In contrast to attenuated bacteria created by spontaneous mutations, attenuated bacteria created by mutations such as deleting fragments of the genes or deleting complete genes or insertion of heterologous DNA-fragments or combinations thereof have the advantage that they will not revert to the wild-type pathogenic bacteria. Accordingly, in a preferred embodiment the invention provides live attenuated bacteria in which at least one gene comprises an insertion and/or a deletion.

In the context of the present invention, the term "nonfunctional" is understood as the blocking of the expression of a specific gene or of a protein either through molecular modification or negative regulation of one or both. Molecular modification includes the use of conventional recombinant DNA techniques which in turn include: the substitution of one or several nucleotides, the insertion of one or several nucleotides, the partial or complete deletion of a gene, chemically-induced or radiation-induced disruption by mutagenesis. Negative regulation of the expression of a gene or protein includes transcriptional and post-transcriptional gene silencing.

The term "nonfunctional" is understood as any bacterial strain unable to produce a functional and/or active form of the respective protein. This deficiency can be due to: blocking of the expression of the coding genes thereof, post-transcriptional modifications and post-translational modifications affecting enzymatic activity, allosteric regulation or the cellular location of this enzyme.

In another embodiment the *Escherichia Coli* of present invention, wherein the *Escherichia Coli* is a mutant live cell wall auxotrophic bacterial strain. In the context of the present invention, the term "auxotrophic" is understood as the lack of a functional metabolic pathway, on which the thus designated bacterium depends for growth, due to the inability to synthesize a specific compound.

In another embodiment the *Escherichia Coli* of present invention, wherein the mutation is a mutation in asd gene, or a mutation in alr, metC and dadX gene or a mutation in the murl gene, or a mutation in asd, alr, metC and dadX gene, or a mutation in alr, metC, dadX and murl gene. In a preferred embodiment the mutation is in the asd, alr, metC, dadX and murl gene.

In another embodiment the *Escherichia Coli* of the present invention, wherein the attenuated *Escherichia Coli* can persist in a subject. As used herein the terms "persist" and "persistent" refer to the establishment and/or maintenance of an infection or colonisation of at least part of the subject. Persistence includes a state in which an organism survives in tissues or on body surfaces / in association with body surfaces, whether replicating or not. Persistent may or may not be associated with clinically relevant disease.

In a preferred embodiment the live attenuated bacteria persist in the subject. Persistence of the attenuated bacteria is preferably assessed in a subject-disease model system but may also be assessed in subjects to which the vaccine composition or immunogenic composition described herein have been administered. Persistence of the attenuated bacteria may be assessed by way of observation of clinical signs and/or symptoms. Alternatively or in addition persistence may be assessed by determining the presence of the attenuated bacterium in a sample taken from the subject. The sample may be a tissue sample (e.g. blood, skin, hair, buccal scraping) or a sample of bodily secretion or excretion for instance mucus, urine, faeces, lacrimal fluid. Samples may be taken while the subject is alive or at autopsy or necropsy. The presence of the attenuated bacteria in the sample may be assessed by culture, molecular methods such as ELISA or PCR or any method known in the art. Furthermore, observation of affected regions of the subject at autopsy or necropsy may also allow determination that the attenuated bacterium persists in the subject.

In another embodiment the *Escherichia Coli* is extraintestinal *E. Coli,* preferably is Avian Pathogenic *Escherichia Coli* (APEC). Avian pathogenic *E. coli* (APEC) comprises a specific subset of pathogenic *E. coli* that cause extraintestinal diseases of poultry. APEC consists mainly of enteropathogenic *E. coli* (EPEC) and enterotoxigenic *E. coli* (ETEC) serovars, i.e., subdivisions of a species or subspecies distinguishable from other strains therein on the basis of antigenicity. APEC is an ubiquitously present pathogen that can also be found in the intestinal microflora of healthy birds. APEC can cause either primary infections or secondary ones that are enhanced or initiated by environmental and host predisposing factors.

In another embodiment the mutation is generated by insertion, deletion, substitution or any combination thereof, preferably the mutation is a deletion of the gene.

In the context of the present invention, the term "air gene" is understood as a gene or nucleotide sequence encoding an alanine racemase protein.

In the context of the present invention, the term "asd gene" is understood as a gene or nucleotide sequence encoding a aspartate semialdehyde dehydrogenase protein.

In the context of the present invention, the term "metC gene" is understood as a gene or nucleotide sequence encoding a cystathionine beta- lyase protein.

In the context of the present invention, the term "dadX gene" is understood as a gene or nucleotide sequence encoding a alanine racemase protein.

In the context of the present invention, the term "murl gene" is understood as a gene or nucleotide sequence encoding a glutamate racemase protein.

In another embodiment the Escherichia Coli is Avian Pathogenic Escherichia Coli strain (SOP017.1), deposited under the code DSM 35079. Herein said bacterial strain is the bacterial strain of *E. Coli* designated DSM 35079 and deposited under the Budapest treaty before the Leibniz Institute DSMZ-German Collection of Microorganisms and Cell Cultures on 24 June 2024.

The invention provides the avian pathogenic *Escherichia coli* inactivated vaccine with the deposit number DSM 35079. Accordingly, an avian pathogenic *Escherichia coli* inactivated vaccine deposited under Leibniz Institute DSMZ-German Collection of Microorganisms and Cell Cultures accession number DSM 35079 or a derivative thereof is provided herein.

In another embodiment the *E. Coli* of the present invention comprises SEQ ID NO: 1, 2, 3, 4, or 5. In another embodiment the *E. Coli* of the present invention comprises SEQ ID NO: 1 and 2. In another embodiment the *E. Coli* of the present invention comprises SEQ ID NO: 1, 2 and 3. In another embodiment the *E. Coli* of the present invention comprises SEQ ID NO: 1, 2, 3 and 4. In another embodiment the *E. Coli* of the present invention comprises SEQ ID NO: 1, 2, 3, 4 and 5.

In another embodiment the subject is a vertebrate animal. The vertebrate animal may be selected from the group comprising humans, bovines, canines, felines, caprines, ovines, porcines, camelids, equines and avians. The avian subject may be any commercially or domestically raised avian. The avian may be selected from the group comprising chickens (including bantams), turkeys, ducks, geese, pheasants, quails, partridges, pigeons, guinea-fowls, ostriches, emus or pea-fowl. Still, most particularly, the pathogenic bacteria stain is an APEC and the livestock is poultry, such as broiler chicks.

In another aspect the present invention provides an immunogenic composition comprising the *Escherichia Coli* according to the invention.

In another aspect the present invention provides a vaccine composition comprising an immunogenically effective amount of at least one *Escherichia Coli* of the present invention and a pharmacologically acceptable carrier. The pharmaceutically acceptable carrier or diluent is selected from the list consisting of water, culture fluid, a solution of physiological salt concentration and/or stabilisers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer).

In one embodiment the immunogenic composition further comprises a commensal bacteria. Commensal bacteria are types of bacteria that live on or within a host organism in a manner where the host is neither harmed nor benefited. The commensal bacteria in the present invention may be selected from the phylum comprising Firmicutes, Bacteroidetes and Actinobacteria.

In one embodiment the vaccine compositions or immunogenic compositions are lyophilized or freeze-dried.

In another embodiment the vaccine composition or immunogenic composition further comprising at least one adjuvant. The adjuvant may be selected from the group comprising Freund's complete adjuvant, Freund's incomplete adjuvant, vitamin E, non-ionic block polymers, muramyldipeptides, saponins, mineral oil, vegetable oil, carbopol aluminium hydroxide, aluminium phosphate, aluminium oxide, oil-emulsions saponins, vitamin- E solubilisate or any combination thereof.

In some embodiments the vaccine composition or immunogenic composition may comprise at least one compound having adjuvant activity. Examples of adjuvants suitable for use in vaccine compositions may be selected from the group comprising Freund's complete or Freund's incomplete adjuvant, vitamin E, non-ionic block polymers, muramyldipeptides, saponins, mineral oil, vegetable oil, carbopol aluminium hydroxide, aluminium phosphate, aluminium oxide, oil-emulsions (e.g. of Bayol F^{®} or Marcol 52^{®}), saponins or vitamin-E solubilisate or any combination thereof. In some embodiments the vaccine composition or immunogenic composition may comprise adjuvants particularly useful for mucosal application for example *E*. *coli* heat-labile toxin or Cholera toxin.

In another embodiment the vaccine composition or immunogenic composition is administered intranasally, opthalmically, intradermally, intraperitoneally, intravenously, subcutaneously, orally, cloacally, by aerosol (spray vaccination) or intramuscularly. In one embodiment the E coli of the present invention may be administered by any conventional means, preferably an economically viable means for the poultry industry such as mass administration via spray or drinking water.

In another embodiment the vaccine composition or immunogenic comprises at least about 10³ to about 10⁵ attenuated bacteria, or about 10⁵ to about 10⁷ attenuated bacteria, or 10⁵ to about 10¹¹ attenuated bacteria or about 10⁷ to about 10⁹ attenuated bacteria, or about 10⁹ to about 10¹¹ attenuated bacteria, or about 10¹¹ to about 10¹³ attenuated bacteria, or about 10¹³ to about 10¹⁵ attenuated bacteria, or about 10¹⁵ to about 10¹⁷ attenuated bacteria, or about 10¹⁷ to about 10¹⁹, or at least about 10¹⁹ attenuated bacteria per dose.

In another embodiment in addition to the *E .coli* of the present invention as an active ingredient, it is contemplated the vaccine composition or immunogenic composition of the invention may also contain other active components such as an avian immunogenic antipathogenic compound directed against avian leukosis, reticuloendotheliosis, infectious bronchitis, infectious bursal disease, Newcastle disease, avian adenovirus disease, avian reovirus disease, fowl pox disease, infectious laryngotracheitis, avian influenza, infectious coryza, fowl typhoid, coccidiosis, cryptosporidiosis, fowl cholera, or the like.

In another aspect the present invention provides a method for attenuating *Escherichia Coli,* the method comprising mutating at least one alr, asd, metC and dadX and murl gene and wherein the E. col of the present invention persists in a subject.

In another aspect the present invention provides a method of prevention or amelioration of a disease in a subject, the method comprising administering a therapeutically effective dose of a vaccine composition or immunogenic composition according to the present invention to the subject.

In the context of the present invention, the expression "therapeutically effective dose" refers to the amount of attenuated bacterial strains of the invention that allow producing the desired effect.

The pharmaceutically acceptable adjuvants and carriers that can be used in said compositions are carriers known by persons skilled in the art. The compositions provided by this invention can be facilitated through any administration route, for which purpose said composition will be formulated in the suitable dosage form and with the excipients that are pharmacologically acceptable for the chosen administration route.

The terms "inhibiting," "reducing," or "prevention," or any variation of these terms, when used in the claims and/or the specification includes any measurable decrease or complete inhibition to achieve a desired result.

Immunogenically effective amounts may vary according to the age and size of the host, the severity of the infection, the virility of the pathogen, the mode of administration or the like. In general, suitable effective amounts per dosage unit may be about 10² to 10¹⁴ colony forming units (cfu), preferably about 5.0 ×10² to 5.0 ×10¹⁰ cfu, more preferably about 3.0 ×10⁶ cfu to 6.0 ×10⁶ cfu, and even more preferably about 5.0 ×10⁶ cfu to 6.0 ×10⁶ cfu. One or two dosage units may be contemplated by the skilled artisan. If two dosage units are selected, then vaccination at about day 1 post-hatch and again at about one week to two weeks of age is preferred. A dosage unit is desirably about 0.5 to 1 mL of vaccine per bird, but that quantity may be optimized to deliver an immunogenically effective amount of the microorganism hereinabove described.

The vaccine or immunogenic composition of the invention according to the embodiments herein described is to be considered efficacious against all serotypes of *E .coli* colibacillosis, including serotypes O1, O2 and O78, as well as the especially virulent untyped serotypes. The vaccine herein described is efficacious against septicemia, pericarditis, airsacculitis, periphepatitis, arthritis, and particularly cellulitis. The latter is often associated with colibacillosis, but can be a significant problem in itself.

In another aspect, the present invention provides a method of prophylaxis of a disease, the method comprising administering a therapeutically effective dose of a vaccine composition or immunogenic composition according to the preset invention to a subject in need of prophylaxis.

In another aspect, the present invention provides the use of a vaccine composition for the treatment or prophylaxis of disease wherein the vaccine composition comprises at least one *Escherichia Coli* of the present invention. The experimental examples described herein provide procedures and results which establish that auxotrophic bacterial strains of the present invention are sufficiently a-virulent (attenuated) to avoid unacceptable pathological effects, induce a sufficient level of immunity in the host independently of the administration route and have a substantial level of security (both environmental and for the host) for their use in active or passive immunization.

Those skilled in the art will appreciate that the invention described herein is susceptible to variations and modifications other than those specifically described. It is to be understood that the invention includes all such variation and modifications. The invention also includes all of the steps, features, compositions and compounds referred to or indicated in the specification, individually or collectively and any and all combinations or any two or more of the steps or features.

The present invention is not to be limited in scope by the specific embodiments described herein, which are intended for the purpose of exemplification only. Functionally equivalent products, compositions and methods are clearly within the scope of the invention as described herein.

The invention contemplates combinations of any of the foregoing aspects and embodiments of the invention. Each and every embodiment described throughout the application can be combined, and can be applied to each and every aspect of the invention described herein. Further, the methods and reagents of the various aspects of the instant invention can be used prophylactically. Alternatively, they can be used therapeutically. The present invention is further illustrated by the figures and examples from which further features, embodiments and advantages may be taken.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

For a more clear understanding of the invention, the following examples are set forth below. These examples are merely illustrative and are not understood to limit the scope or underlying principles of the invention in any way. Indeed, various modifications of the invention, in addition to those shown and described herein, will become apparent to those skilled in the art from the examples set forth herein below and the foregoing description. Such modifications are also intended to fall within the scope of the appended claims.

### Examples

### Materials and Methods

### Bacterial strains and culture media

The bacterial strains and plasmid used in this study are listed in table 1. Genes were knocked out using the Lambda Red recombineering leading to clean knock outs or gene replacements with an antibiotic resistance cassette. Due to enhanced sucrose sensitivity of pathogenic *E. coli* strains we used TSB for counter selection instead of LB.

Tryptic soy broth and agar (TSB/TSA) (BD Difco) was used as the standard medium for liquid and solid cultures. MacConkey II (Oxoid) agar was used for solid cultures with agarose 1.5% w/v. L-from like agar (LFLA) had BHIA as its base and was supplemented with saccharose (Sigma, 0.3M) and MgSO4 7 H2O (Sigma, 0.1M). Chromagar Orientation (Chromagar). Where necessary and appropriate, the media were supplemented with Ampicillin (Sigma; 100mg/l), Tetracycline (Sigma; 12.5 mg/l), meso-diaminopimelic acid (Sigma; 50mg/l) or D-alanine (200mg/l, all purchased from Sigma).

### Cell culture adhesion assay

HeLa cells (Kyoto) were grown in Dulbecco modified Eagle's medium (DMEM, Gibco) with 10% fetal bovine serum (FBS, Sigma) and incubated at 37°C at 5% CO₂. For cellular invasion assay cells were seeded in 24 well plates and grown until 80% confluency was reached. Prior to infection with different *E. coli* strains the cells were incubated with Hanks' balanced salt solution (HBSS, Gibco) supplemented with D-Ala and m-DAP (for auxotrophic *E. coli* strains). *E. coli* strains were cultured in TSB and diluted in HBSS to approximately 106 CFU/well (cultured on solid medium for exact count) to infect the HeLa cells. The cells were incubated for 15min at 37°C at 5% CO₂. After infection the cells were vigorously washed with PBS 3 times. After washing cells were lysed with PBS + 0.1% sodiumdesoxy-cholat and the suspension cultured on solid medium to determine the attached bacterial count.

### Animal experiments

Animal experiments were performed in accordance to animal experiment license (BE95_2022 & BE108_23) approved by the Bernese Cantonal Ethical committee for animal experiments and carried out in accordance with Swiss Federal law for animal experimentation. Chickens were maintained under specific pathogen free conditions at the Institute for Virology and Immunology of the Federal Food Safety and Veterinary Office (FSVO). The ambient temperature was 23-25°C and a warming lamp was provided at all times. All animals received at all times standard diet and water ad libitum. Infection and colonization experiments were performed under strict aseptic conditions. Chickens were kept per treatment group in separate sealed rooms where experimenters entered via airlock and changed clothes in between rooms. Animals were kept in these rooms for the duration of the experiment and were transferred to a dedicated slaughter hall for terminal sampling.

Chickens were crop inoculated with a specified amount of bacterial strains. For this purpose the strains were grown in adequately sized liquid culture without antibiotics. Cultures were washed in PBS and diluted to the desired density. The chicken received at all times a volume of 200µl of inoculum.

Health of chickens was assessed daily during experiments by measuring body weight (balance Prima Vista, Landi) and body temperature with an ordinary fever thermometer rectally (Landi). Additionally, the chickens were scored according to predefined parameters displayed in table 2.

### Bacterial loads

Bacterial loads were determined from homogenized samples. Prior to homogenization the net weight of feces was determined using a balance. Feces were homogenized with PBS by vortexing for a few seconds. Organs were homogenized in PBS 1X + 0.05% Tergitol in a Bullet Blender BBX24B at max speed for 3min. Colony forming units of samples were assessed by culturing on MacConkey agar plates with adequate supplementation and appropriate dilution of the sample. IgA titer

In a U-bottom 96 well plate 10⁷ CFU per well of bacteria were seeded. In parallel, serial dilutions of each intestinal wash were prepared and then applied to the bacteria in equal amounts. The antibodies in the intestinal washes were allowed to bind for 30 min on ice. The plate was washed with PBS+BSA 2%. Next, anti-chicken-lgA FITC antibody (Biorad) was diluted 1:200 and applied to each well and incubated for 1h on ice. Plate was washed with PBS+BSA 2% and bacteria were resuspended in 150µl of PBS+PFA 2% for subsequent analysis in a Cytek Aurora Spectral Analyzer in order to determine the antibody coating of the bacteria. For each bacterium the mean fluorescence intensities of each dilution were read out using the software FlowJo and plotted against the actual amount of IgA (obtained by ELISA abcam kit) in the respective wash. The EC50 for each wash was calculated and inversely plotted to show each animal's individual IgA titer.

### Antibiogram

Antibiogram was done according to EUCAST and CLSI. It was tested for a total of 31 clinically relevant antibiotics.

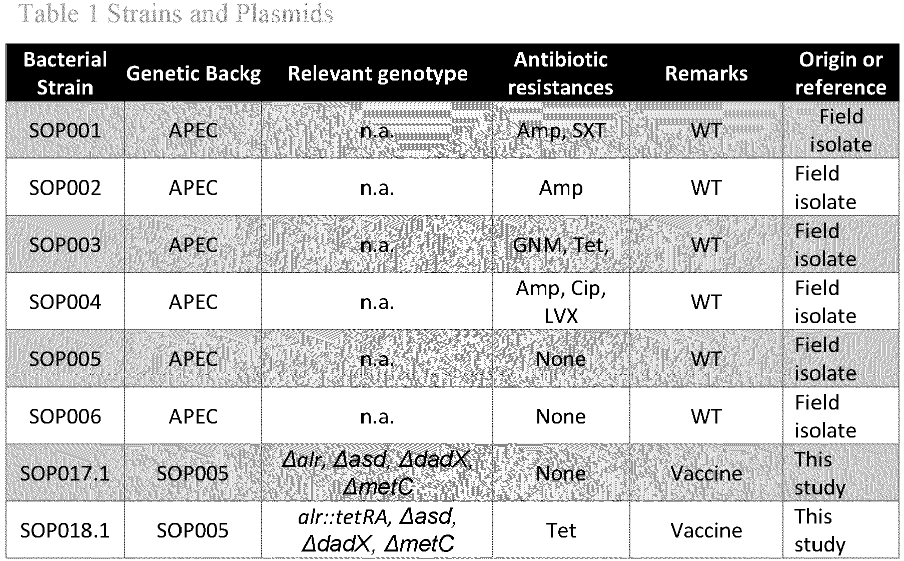

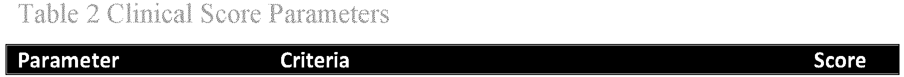

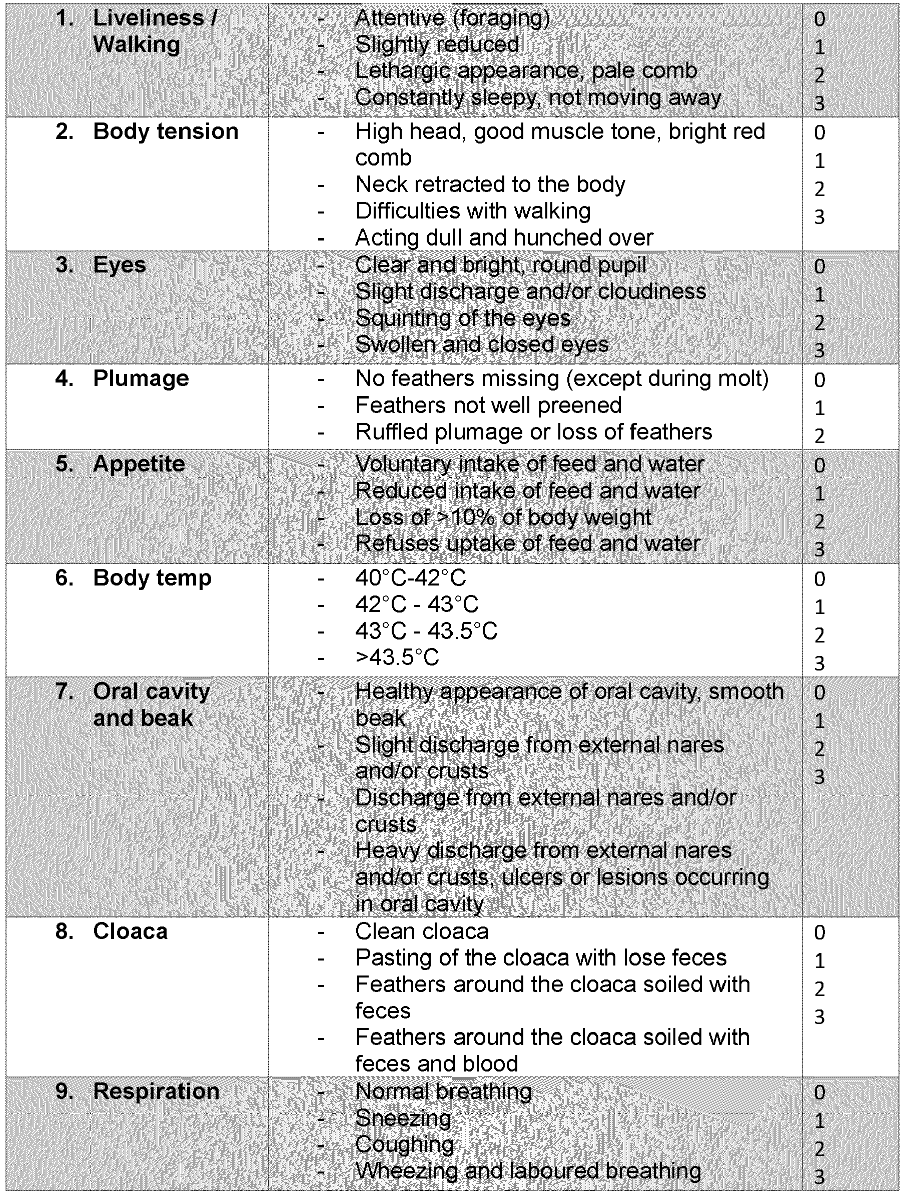

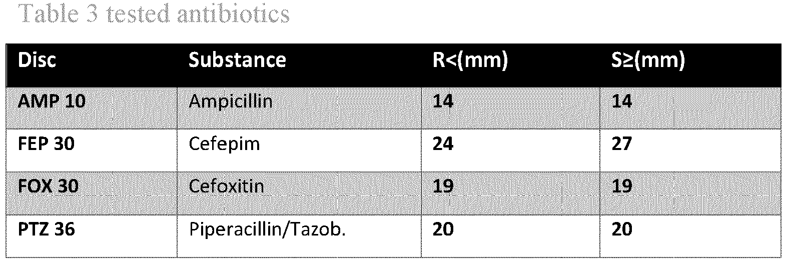

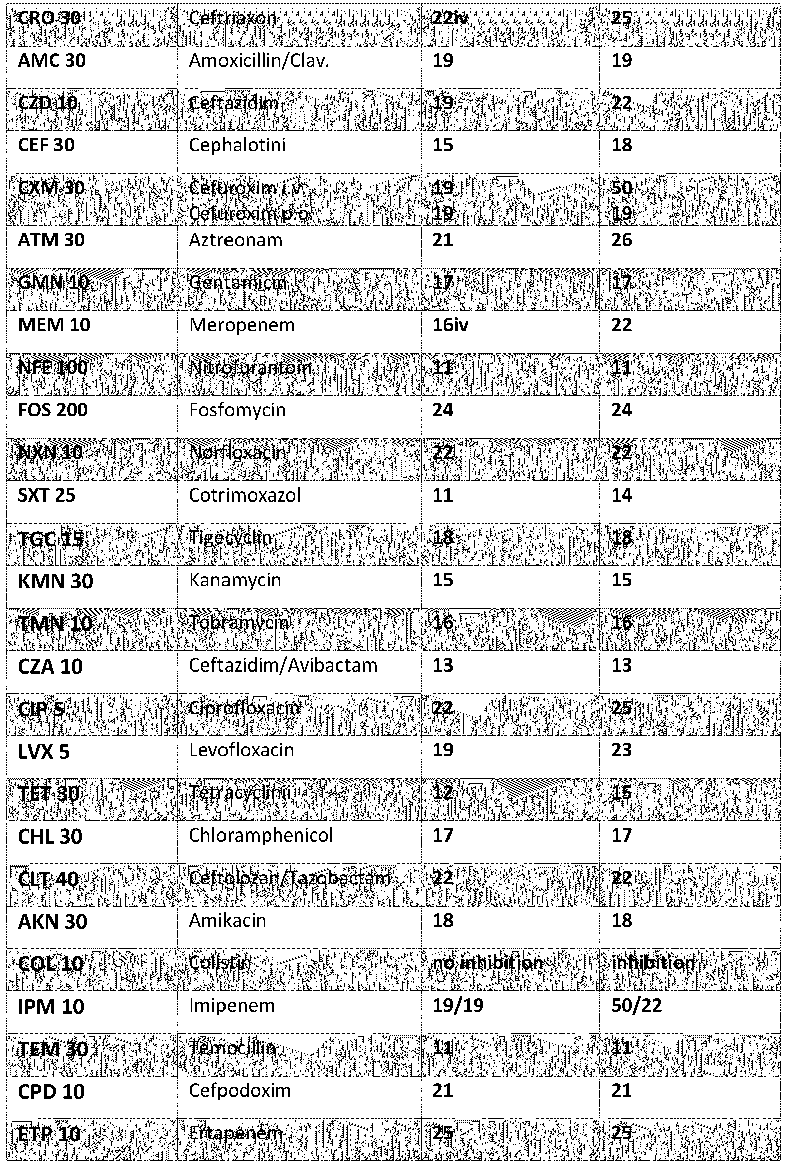

### Isolation of APEC strain

To translate this concept into the epizootic avian pathogenic *E. coli* (APEC) that is very relevant in the poultry industry we had to isolate the relevant strains. We received samples of fecal matter from a Bavarian poultry business where a significant number of animals had succumbed to the infection with APEC. Using classical microbiological methods, we isolated 6 strains of APEC from the samples.

The strains were verified using MALDI-TOF. These were subsequently tested for antibiotic resistances according to EUCAST and CLSI (see table 3). Strains were then in vitro functionally tested for their virulence by PCR and using an adhesion assay. Strain SOP005 was deemed suitable and selected for mutagenesis. The genes alr, asd, metC and dadX were cleanly knocked out of the genome as described in the methods section. Knock outs were confirmed via PCR and sanger sequencing. The strain was named SOP017.1. For the following experimental procedures, the strain was equipped with a tetracycline resistance cassette at the locus of air in order to be able to selectively culture it and distinguish it from endogenous *E. coli* strains. This strain was named SOP018.1.

### Safety of cell wall auxotrophic APEC field isolates

The safety and stability of the cell wall auxotrophic APEC strain SOP018.1 was first assessed in vitro. Information was gained by cultivating SOP018.1 on L-form like (LFL) agar. This agar has a high osmolarity which reduces the osmotic pressure on bacteria growing on it. Bacteria can grow on this medium even without building up a proper cell wall and still do not burst. This medium is ideal to provoke revertants in cell wall auxotrophic strains. We observed revertants at a low frequency for a single compound while supplemented with the other. Escapees to D-Ala (medium + m-DAP) were 100 times more frequent than to m-DAP (medium + D-Ala. Interestingly, revertants escaping the need for both, D-Ala and m-DAP were equally likely to occur as escapees to m-DAP. Morphologies of the observed bacteria were either coccoid or elongated rods highlighting the bacteria's struggle to build up a functional and intact cell wall. Subsequent cultivation of these clones in a non-high-osmolarity medium (i.e. TSB) did not allow for growth, neither on solid nor in liquid medium. The occurrence of revertants capable of surviving in an ecologically relevant in vivo setting was thus deemed very unlikely. An animal experiment to test for the ability to engraft and permanently colonize the niche at two different application regimens was performed. A total of three groups with 10 animals each was used in this experiment. Group 1 served as a control and only received vehicle (i.e. PBS 1X). Groups 2 and 3 received the vaccine strain SOP018.1. While group 2 received three high doses (i.e. 10¹⁰ CFU per animal) on days 0, 14 and 21 post hatching (Fig 1 A) group 3 received a single moderate dose (i.e. ~10⁸ CFU per animal) .

The safety of strain SOP018.1 was assessed by measuring the body weight (Fig1 B), the body temperature (Fig1 C) as well as assessing a clinical score daily. Comparing all groups there was no significant difference in terms of body weight and its increase and body temperature. One can appreciate that the body temperature is not very stable during the first few days post hatching but stabilizes at day 10 post hatching.

Niche engraftment and colonization capabilities of SOP018.1

We monitored the density of SOP018.1 daily to assess how well the vaccine strain is able to engraft the niche and if at all persists in it by taking cloacal swabs. This is the only non invasive method that allows for continued sampling of fecal matter. It being the end of the excretory system it gives an approximation of what is actually happening in the chicken's GI tract.

The fecal densities of SOP018.1 in the chickens from groups 2 and 3 are shown in Fig 2 A. Group 2 (black asterisks) received high doses within 12h after hatching as well as on days 14 and 21 as a booster. The rationale behind this strategy was to have a lot of the vaccine strain at the earliest possible time point in order to enable it an optimal head start towards the normal microbiota. It is a well-known concept that in ecological niche engraftment settings the first colonizer has an advantage over subsequent and similar yet to this specific environment new colonizers. The big question here being if this notion also applies to this heavily, metabolically impaired strain that will have to compete against very fit endogenous *E. coli.* It is clear that SOP018.1 very efficiently colonizes the respective niche and is also able to persist in there for at least the duration of 42 days which is the maximal normal lifespan of a broiler chicken. This means that there is at least a 5 days window where the chicken can be vaccinated with a variable dose of the vaccine which leads to the desired outcome.

When analysing the different sections of the chicken's GI tract (i.e. duodenum, jejunum, ileum, caeca, cloaca) it becomes apparent that the strain has its difficulties in colonizing under suboptimal nutrient conditions. When comparing it to levels of endogenous *E. coli* in the other groups one can see that not all parts of the GI tract are equally high colonized by *E. coli.* The levels in the duodenum are overall lower per gram of content compared to the other sections. In the ileum, jejunum and the cloaca we can observe remarkable differences between endogenous *E. coli* and SOP018.1 except for the two caeca where the levels are equal (Fig 2 B with schematic of chicken GI tract). Further evidence is given by plotting the competitive indices of SOP018.1 / endogenous *E. coli* both from the same individual. The ratio of the vaccine strain to the endogenous *E. coli* are well balanced in the caeca but are otherwise more in favour of the endogenous *E. coli* strains.

Immunogenicity of cell wall auxotrophic APEC field isolates.

The comparison of immunogenicity of the two administration regimens was an important readout. The entire GI tract of the chicken (from ventriculus to cloaca) was flushed with PBS in order to measure this. Then the concentration of IgA in each wash was determined via ELISA. This was done on day 14 with the surplus animals as well as on day 42 at the endpoint of the experiment. One can nicely see that the overall amount of IgA in the animals is significantly lower in the younger animals indicating that generally the immune system needs to mature in order to produce the necessary amounts of IgA. Since the total amount of IgA in an animal does not indicate about the immune status for a certain antigen these values were then later used to normalize the titer. To determine the specificity of IgA against the vaccine we measured the affinity of each intestinal wash against its respective wild type pathogen. One can appreciate the massive difference in the normalized affinities against its respective wild type pathogen. The highest titer can be observed in the boosted group having received high doses followed by the group having received a single dose of SOP018.1 on day 5.

### Discussion

In this study, we demonstrate the safety and effectiveness of a genetically modified probiotic vaccine derived from a wild-type pathogen. To assess the safety of the vaccine, we evaluated the well-being of treated animals by measuring relevant parameters and compared them to an untreated control group. Our results showed no significant differences between the treated and control animals, indicating that the vaccine is safe to use and has no negative impact on animal development. These findings are particularly surprising for animals that received a high dose of the vaccine within the first 12 hours after hatching, as they are naive to resident microbiota and therefore vulnerable to bacterial infections.

Despite the potential risks associated with introducing an artificially high amount of metabolically impaired but still virulent bacteria into the animals, our vaccine strain was well-tolerated by the chicks, highlighting its unsurpassed safety. Additionally, the vaccine strain proved to be a stable and effective colonizer of the chicken gut, even in the presence of competing bacteria that establish in the course of the chicken's life. This indicates that the vaccine may provide early protection against APEC infections through enhanced colonisation resistance, even before the chicken's immune system is fully mature.

Moreover, our results suggest that the vaccine strain is highly immunogenic, as we detected robust and specific IgA antibodies against the respective wild-type strain at the end of the experiment on day 42. Taken together, these findings strongly suggest that the vaccine strain SOP018.1 is a promising candidate for protecting chickens against APEC infections.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent, or similar purpose, unless expressly stated otherwise. Thus, unless expressly stated otherwise, each feature disclosed is one example only of a generic series of equivalent or similar features.

The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

### Sequences

Seq ID NO: 1 (Alr) >E. coli APEC SOP005 alanine racemase 
ATGCAAGCGGCAACTGTTGTGATTAACCGCCGCGCTCTGCGACACAACC
TGCAACGTCTTCGTGAACTGGCGCCTGCCAGTAAAATGGTTGCGGTGGTGAAA
GCGAACGCTTATGGTCACGGTCTTCTTGAGACCGCGCGAACGCTCCCCGATGC
TGACGCCTTTGGCGTAGCCCGTCTCGAAGAAGCTCTGCGACTGCGTGCGGGG
 
GGAATCACCAAACCTGTACTTTTACTCGAAGGCTTTTTTGATGCCAGAGATCTAC
CGACGATTTCTGCGCAACATTTTCATACCGCCGTGCATAACGAAGAACAGCTGG
CTGCGCTGGAAGAGGCTAGCCTGGACGAGCCGGTTACCGTCTGGATGAAACTC
GATACCGGTATGCACCGTCTGGGCGTAAGGCCGGAACAGGCTGAGGCGTTTTA
TCATCGTCTGACCCAGTGCAAAAACGTTCGTCAGCCGGTGAATATCGTCAGCCA
TTTTGCGCGCGCGGATGAACCAAAATGCGGCGCAACCGAGAAGCAACTCGCTA
TCTTTAATACCTTTTGCGAAGGCAAACCTGGTCAACGTTCCATTGCCGCGTCGG
GTGGCATTTTACTCTGGCCACAGTCGCATTTTGACTGGGTGCGCCCGGGCATC
ATTCTTTACGGCGTATCTCCGCTGGAAGATCGCTCCACCGGTGCCGATTTTGGC
TGTCAGCCAGTGATGTCGCTAACCTCCAGCCTGATTGCCGTGCGTGAGCATAAA
GCCGGAGAGCCTGTCGGTTATGGTGGAACCTGGGTAAGCGAACGTGATACTCG
TCTTGGCGTAGTCGCGATGGGCTATGGCGATGGTTATCCGCGCGCCGCGCCGT
CCGGTACGCCAGTGCTGGTGAACGGTCGCGAAGTGCCGATTGTCGGGCGCGT
GGCGATGGATATGATCTGCGTAGACTTAGGTCCACAGGCGCAGGATAAAGCCG
GGGACCCGGTCATTTTATGGGGCGAAGGTTTGCCCGTAGAACGTATCGCTGAA
ATGACGAAAGTAAGTGCTTACGAACTTATCACGCGCCTGACCTCAAGAGTCGCG
ATGAAATACGTGGATTAA
Seq ID NO: 2 (Asd) >E. coli APEC SOP005 aspartate semialdehyde dehydrogenase 
TTACGCCAGTTGACGAAGCATCCGACGCAGCGGCTCCGCGGCCCCCCA
CAGCAGCTGGTCGCCCACGGTAAAGGCTGACAGGAACTCCGGTCCCATATTCA
GCTTACGCAGGCGGCCTACCGGCGTGGTCAGCGTGCCGGTAACGGCAGCTGG
GGTTAGCTCACGCATAGTGATTTCCCGATCGTTCGGAACAACTTTCGCCCACGG
ATTGTGCGCAGCCAGCAGTTCTTCCACGGTCGGAATAGACACATCTTTTTTCAAT
TTAATAGTGAATGCCTGGCTGTGGCAGCGCAATGCCCCGACACGCACACATAAA
CCATCTACCGGAATTACGGAAGATGTGTTGAGGATCTTGTTGGTTTCCGCCTGC
CCTTTCCACTCTTCGCGGCTCTGACCGTTATCAAGCTGTTTGTCGATCCACGGA
ATCAGGCTACCCGCCAGCGGCACGCCAAAGTTATCTACCGGCAGCTCACCGCT
ACGGGTTAAGGTTGTGACTTTGCGTTCGATATCGAGAATAGCAGAGGACGGGTT
CGCGAGTTCATCTGCCACATGGCCATACAGATGGCCCATTTGGGTTAATAACTC
ACGCATATGTCGCGCACCACCGCCGGAAGCGGCCTGGTAGGTTGCAACGGAC
ACCCAATCAACAAGATCATTGGCGAATAAACCCCCCAGCGACATCAACATCAGG
CTTACGGTACAGTTACCGCCAACAAAAGTCCTGATACCATTATTTAATCCGTCGG
TAATGACGTCCTGATTGACGGGGTCAAGAATGATGATGGCGTCATCTTTCATGC
GCAGAGATGATGCTGCGTCAATCCAGTAACCTTGCCATCCGCTTTCACGAAGCT
TTGGATAGATTTCGTTGGTATAATCGCCGCCCTGACAGGTCACAATGATATCGA
 
GGGCCTTTAGCGCCTCCAGATCAAAGGCATCCTGAAGTGTGCCAGTGGTTCCG
CCAAAAGACGGCGCAGCCTGACCAAGCTGAGAAGTAGAAAAGAAGACAGGGCG
AATGGCGTCGAAGTCGCGCTCTTCAACCATGCGTTGCATGAGAACGGAGCCGA
CCATACCGCGCCAGCCGATAAAACCAACATTTTTCAT
Seq ID NO: 3 (MetC) >E. coli APEC SOP005 Cystathionine beta-lyase 
ATGGCGGACAAAAAGCTTGATACTCAACTGGTGAATGCAGGACGCAGCA
AAAAATACACGCTCGGTGCGGTAAATAGCGTGATTCAGCGCGCGTCTTCGCTG
GTCTTTGACAGTGTGGAAGCCAAAAAACACGCGACACGTAATCGCGCCAATGG
CGAGTTGTTCTATGGACGGCGTGGAACGTTAACCCATTTCTCCTTACAACAAGC
GATGTGTGAACTGGAAGGTGGCGCAGGCTGTGTGCTATTTCCCTGCGGGGCGG
CAGCGGTTGCTAATTCCATTCTTGCTTTTGTCGAACAGGGCGATCATGTGTTGAT
GACCAACACCGCCTATGAACCGAGTCAGGATTTCTGTAGCAAAATCCTCAGCAA
ACTGGGCGTAACGACATCGTGGTTTGATCCGCTGATTGGTGCCGATATCGTTAA
TCATCTGCAGCCAAACACTAAAATCGTGTTTCTGGAATCGCCAGGTTCCATCAC
CATGGAAGTCCACGATGTTCCGGCGATTGTTGCCGCCGTACGCAGTGTGGTCC
CGGATGCCATCATTATGATCGACAACACCTGGGCAGCCGGTGTGCTGTTTAAG
GCGCTGGATTTTGGCATCGATGTTTCTATTCAGGCCGCCACCAAATATCTGGTT
GGGCATTCAGATGCGATGATTGGCACTGCCGTGTGCAATGCCCGTTGCTGGGA
GCAGCTGCGAGAGAATGCCTATCTGATGGGCCAGATGGTCGATGCCGATACCG
CCTATATAACCAGCCGTGGCCTGCGCACTTTGGGGGTTCGCCTGCGTCAACAT
CATGAAAGCAGTCTGAAAGTGGCTGAATGGCTGGCAGAACATCCCCAAGTAGC
ACGAGTTAACCACCCTGCTCTGCCTGGCAGTAAAGGATACGAATTCTGGAAACG
AGACTTTACAGGCAGCAGCGGGCTATTTTCCTTTGTGCTTAAGAAAAAACTCAAT
GATGAAGAGCTGGCGAACTATCTGGATAACTTCAGTTTATTCAGCATGGCCTAC
TCGTGGGGCGGGTATGAATCGTTGATCCTGGCAAATCAACCAGAACATATCGCC
GCCATTCGCCCACAAGGCAAGATCGATTTTAGCGGGACCTTGATTCGCCTGCAT
ATTGGTCTGGAAGATGTCGACGATCTGATTGCCGATCTGGACGCCGGTTTTGCG
CGAATTGTATAA
Seq ID NO: 4 (DadX) >E. coli APEC SOP005 alanine racemase, catabolic 
ATGACCCGTCCGATACAGGCCAGCCTCGATCTGCAGGCATTAAAACAGA
ATCTGTCCATTGTCCGCCAGGCCGCGCCGCGCGCGCGCGTCTGGTCGGTGGT
AAAAGCGAACGCTTACGGGCACGGTATTGAGCGTATCTGGAGCGCGATCGGGG
CTACCGATGGCTTTGCATTGCTTAACCTGGAAGAGGCAATAACGTTACGTGAGC
GCGGCTGGAAAGGACCGATCCTGATGCTGGAAGGATTTTTCCATGCTCAGGAT
CTGGAGATTTATGACCAGCACCGCCTGACCACCTGCGTACACAGCAACTGGCA
 
GCTCAAAGCACTGCAAAATGCGCGGCTAAAAGCACCGTTGGATATTTATCTTAA
AGTGAACAGTGGAATGAATCGGTTGGGCTTCCAGCCCGATCGCGTGCTTACCG
TCTGGCAGCAGTTGCGGGCGATGGCGAATGTTGGCGAAATGACCCTGATGTCA
CATTTTGCCGAAGCGGAACATCCTGATGGAATTTCCGGCGCGATGGCGCGTATT
GAGCAGGCGGCGGAAGGGCTGGAGTGTCGGCGTTCGTTGTCCAATTCGGCGG
CGACTCTGTGGCACCCGGAAGCGCATTTTGACTGGGTTCGGCCTGGCATTATTT
TGTATGGCGCTTCGCCGTCCGGTCAGTGGCGTGATATCGCCAATACCGGATTA
CGTCCGGTGATGACGCTAAGCAGTGAGATTATTGGTGTCCAGACGCTAAAAGC
GGGCGAGCGTGTGGGCTACGGCGGTCGCTATACTGCGCGCGATGAACAGCGA
ATCGGCATTGTCGCCGCAGGATACGCCGACGGTTATCCGCGCCACGCGCCTAC
CGGTACCCCTGTTTTAGTGGACGGCGTTCGCACCATGACGGTGGGGACAGTCT
CGATGGATATGCTGGCGGTCGATTTAACGCCTTGCCCGCAAGCGGGTATTGGT
ACGCCAGTTGAGCTGTGGGGCAAGGAGATCAAAATTGATGATGTCGCCGCCGC
TGCCGGAACGGTAGGCTATGAGTTGATGTGCGCGCTGGCGTTACGCGTCCCGG
TTGTGACGGTGTAA
Seq ID NO: 5 (Murl) >E. coli APEC SOP005 glutamate racemase

TCAGCCTAAAACTGCCAGTTTTTCGAGCGTTTCGAAGCCGTAACGCTGT
AAAACGGGCAATAATTGTTCAGCTTCTGGCGTCATTGCCATACAAAAGGCAATAT
TCGCATCGGCAGATTTTGCATCCGGGGCTTCATGTTCTAACAGCCAGGCCGTTC
GGCGAGCAATCGCTGCGCCAGAATCCACCAGCCGGGTTCCCTCTGGCAGCAC
CTGTAACAGTTCTTCTTGTAGTAGAGGGAAATGGGTACACCCCAGTACAACGGT
ATCTGGCGGCTCTTTCATTCTTAACCACGGGCGTAGGATACGTTTTAGCGCATC
CAGAGAAACATCTTCGCCATGCAGCTTCGCTTCAGCCAACTCAACCATCTCTGC
CGAGCCCAGCATTTCTATCTGGCATTCATTAGCGAAACGCGCGATCAGCTCATG
AGTATAAGAACGTTTAACAGTTCCGCGGGTTGCCAGTAATCCGACAATGCCATT
TGCCGTCAGACGTGCAGCAGGTTTAATCGCCGGCACGACACCAACAACCGGGA
AGTCGAACTTTTCGCGTAATGCAGGAAGTGAAACGGTACTGGCGGTGTTGCAA
GCGACCACAGCCAGCGCAAGGGGATAACGTTCTTGCACTGCGGTGACAATTTC
CACCACTCGCTCAACAATAAACACTTCGCTTTTTTCGCCATACGGGAAAGCGAC
GTTATCGAAAGCATAAATGTAATGGAGATCCGGTAAGAGATGCCGGATCTCGTC
ATAGACCGACAACCCACCGACGCCGGAGTCAAACACCAGCACGGTGGGACGT
GGTTCAGAAGGTGTAGCTGCCAGACAAGGTGTATTCCCGTCCTGCAGTTTGGTA
GCCAT

### References

1. Kohanski, M.A., D.J. Dwyer, and J.J. Collins, How antibiotics kill bacteria: from targets to networks. Nature Reviews Microbiology, 2010. 8(6): p. 423-435.
2. Hoiseth, S.K. and B.A.D. Stocker, Aromatic-dependent Salmonella typhimurium are non-virulent and effective as live vaccines. Nature, 1981. 291(5812): p. 238-239.
3. Dodds, D., et al., Controlling the Growth of the Skin Commensal Staphylococcus epidermidis Using D-Alanine Auxotrophy. 2020, Cold Spring Harbor Laboratory.
4. Cabral, M.P., et al., Design of live attenuated bacterial vaccines based on D-glutamate auxotrophy. Nature Communications, 2017. 8: p. 15480.
5. Moscoso, M., et al., A D-Alanine auxotrophic live vaccine is effective against lethal infection caused by Staphylococcus aureus. Virulence, 2018. 9(1): p. 604-620.
6. Germanier, R. and E. Fiirer, Isolation and Characterization of Gal E Mutant Ty 21a of Salmonella typhi: A Candidate Strain for a Live, Oral Typhoid Vaccine. Journal of Infectious Diseases, 1975. 131(5): p. 553-558.
7. Germanier, R., Oral typhoid vaccine and method of preparing the same, U.S.P.A.T. OFFICE, Editor. 1973, Institut Zur Erforschung Der Infektionskrankheiten Cilag GmbH International: United States, p. 7.
8. Hapfelmeier, S., et al., Reversible Microbial Colonization of Germ-Free Mice Reveals the Dynamics of IgA Immune Responses. Science, 2010. 328(5986): p. 1705.
9. Fritz, J.H., et al., Acquisition of a multifunctional IgA+ plasma cell phenotype in the gut. Nature, 2012. 481(7380): p. 199-203.
10. Gomez de Agüero, M., et al., The maternal microbiota drives early postnatal innate immune development. Science, 2016. 351 (6279): p. 1296.
11. Cuenca, M., et al., D-Alanine-Controlled Transient Intestinal Mono-Colonization with Non-Laboratory-Adapted Commensal E. coli Strain HS. PLOS ONE, 2016. 11: p. e0151872.
12. Uchimura, Y., et al., Antibodies Set Boundaries Limiting Microbial Metabolite Penetration and the Resultant Mammalian Host Response. Immunity, 2018. 49(3): p. 545-559.e5.
13. Schären, O.P., et al., Uncoupling of invasive bacterial mucosal immunogenicity from pathogenicity. Nature Communications, 2020. 11(1).
14. Li, H., et al., Mucosal or systemic microbiota exposures shape the B cell repertoire. Nature, 2020. 584(7820): p. 274-278.
15. Romoli, O., et al., Production of germ-free mosquitoes via transient colonisation allows stage-specific investigation of host-microbiota interactions. Nature Communications, 2021. 12(1).
16. Russell, G.A., et al., A protocol for generating germ-free Heligmosomoides polygyrus bakeri larvae for gnotobiotic helminth infection studies. STAR Protocols, 2021. 2(4): p. 100946.
17. Yin, L., et al., The Escherichia coli type III secretion system 2 Is involved in the biofilm formation and virulence of avian Pathogenic Escherichia coli. Comparative Immunology, Microbiology and Infectious Diseases, 2021. 79: p. 101722.
18. Mehat, J.W., A.H.M. Van Vliet, and R.M. La Ragione, The Avian Pathogenic Escherichia coli (APEC) pathotype is comprised of multiple distinct, independent genotypes. Avian Pathology, 2021. 50(5): p. 402-416.
19. Mageiros, L., et al., Genome evolution and the emergence of pathogenicity in avian Escherichia coli. Nature Communications, 2021. 12(1).
20. Kathayat, D., et al., Avian Pathogenic Escherichia coli (APEC): An Overview of Virulence and Pathogenesis Factors, Zoonotic Potential, and Control Strategies. Pathogens, 2021. 10(4): p. 467.
21. Guabiraba, R. and C. Schouler, Avian colibacillosis: still many black holes. FEMS Microbiology Letters, 2015. 362(15): p. fnv118.
22. Ghunaim, H., M.A. Abu-Madi, and S. Kariyawasam, Advances in vaccination against avian pathogenic Escherichia coli respiratory disease: Potentials and limitations. Veterinary Microbiology, 2014. 172(1-2): p. 13-22.
23. Yeoman, C.J., et al., The microbiome of the chicken gastrointestinal tract. Animal Health Research Reviews, 2012. 13(1): p. 89-99.
24. Ducarmon, Q.R., et al., Gut Microbiota and Colonization Resistance against Bacterial Enteric Infection. Microbiology and Molecular Biology Reviews, 2019. 83(3).
25. Morris, J.J., R.E. Lenski, and E.R. Zinser, The Black Queen Hypothesis: Evolution of Dependencies through Adaptive Gene Loss. mBio, 2012. 3(2): p. e00036-12-e00036.
26. Silhavy, T.J., D. Kahne, and S. Walker, The Bacterial Cell Envelope. Cold Spring Harbor Perspectives in Biology, 2010. 2(5): p. a000414-a000414.
27. Clarke, S.C., et al., Virulence of Enteropathogenic <i>Escherichia coli</i>, a Global Pathogen. Clinical Microbiology Reviews, 2003. 16(3): p. 365-378.
28. Landman, W.J.M. and J.H.H. Van Eck, The incidence and economic impact of the Escherichia coliperitonitis syndrome in Dutch poultry farming. Avian Pathology, 2015. 44(5): p. 370-378.
29. Van Der Westhuizen, W.A. and R.R. Bragg, Multiplex polymerase chain reaction for screening avian pathogenicEscherichia colifor virulence genes. Avian Pathology, 2012. 41(1): p. 33-40.
30. Joseleau-Petit, D., et al., Unstable <em>Escherichia coli<lem> L Forms Revisited: Growth Requires Peptidoglycan Synthesis. Journal of Bacteriology, 2007. 189(18): p. 6512-6520.
31. Litvak, Y. and A.J. Bäumler, The founder hypothesis: A basis for microbiota resistance, diversity in taxa carriage, and colonization resistance against pathogens. PLOS Pathogens, 2019. 15(2): p. e1007563.
32. Kincade, P.W. and M.D. Cooper, Development and Distribution of Immunoglobulin-Containing Cells in the Chicken: An Immunofluorescent Analysis Using Purified Antibodies to µ, γ and Light Chains1,2. The Journal of Immunology, 1971. 106(2): p. 371-382.
33. Mast, J. and B.M. Goddeeris, Development of immunocompetence of broiler chickens. Veterinary Immunology and Immunopathology, 1999. 70(3): p. 245-256.
34. Bar-Shira, E., D. Sklan, and A. Friedman, Establishment of immune competence in the avian GALT during the immediate post-hatch period. Developmental & Comparative Immunology, 2003. 27(2): p. 147-157.
35. Ratcliffe, M.J.H. and K.A. Jacobsen, Rearrangement of immunoglobulin genes in chicken B cell development. Seminars in Immunology, 1994. 6(3): p. 175-184.
36. Zhou, Q., et al., The Spatial and Temporal Characterization of Gut Microbiota in Broilers. Front Vet Sci, 2021. 8: p. 712226.
37. Thomason, L.C., et al., Recombineering: Genetic Engineering in Bacteria Using Homologous Recombination. Current Protocols in Molecular Biology, 2014. 106(1): p. 1.16.1-1.16.39.
38. Li, X.-t., et al., Positive and negative selection using the tetA-sacB cassette: recombineering and P1 transduction in Escherichia coli. Nucleic Acids Research, 2013. 41(22): p. e204-e204.

### Numbered Embodiments of the Invention

1. An *Escherichia Coli* attenuated by a mutation in any one of the following genes alr, asd, metC, dadX and murl, wherein the mutation renders the corresponding alr, asd, metC, dadX and murl protein nonfunctional.
2. An *Escherichia Coli* of embodiment 1, wherein the *Escherichia Coli* is a mutant live cell wall auxotrophic bacterial strain.
3. An *Escherichia Coli* of embodiment 1, wherein the mutation is a mutation in asd gene, or a mutation in alr, metC and dadX gene or a mutation in the murl gene, or a mutation in airs, asd, metC and dadX gene, or a mutation in alr, asd, metC, dadX and murl gene.
4. An *Escherichia Coli* of embodiment 1, wherein the attenuated *Escherichia Coli* can persist in a subject.
5. The *Escherichia Coli* of embodiment 1, wherein *Escherichia Coli* is extraintestinal *E. Coli,* preferably is Avian Pathogenic *Escherichia Coli* (APEC).
6. The *Escherichia Coli* of any preceding embodiment, wherein the mutation is generated by insertion, deletion, substitution or any combination thereof, preferably the mutation is a deletion of the gene.
7. The *Escherichia Coli* of any preceding embodiment, wherein the air gene encodes a alanine racemase protein.
8. The *Escherichia Coli* of any preceding embodiment, wherein the asd gene encodes a aspartate semialdehyde dehydrogenase protein.
9. The *Escherichia Coli* of any preceding embodiment, wherein the metC gene encodes a cystathionine beta- lyase protein.
10. The *Escherichia Coli* of any preceding embodiment, wherein the dadX gene encodes a alanine racemase protein.
11. The *Escherichia Coli* of any preceding embodiment, wherein the murl gene encodes a glutamate racemase protein.
12. The *Escherichia Coli* according to embodiments 5-11, wherein the APEC is APEC strain SOP017, deposited under the code DSM 35079 or a derivative thereof.
13. The *Escherichia Coli* of any preceding embodiment, comprising sequence ID NO: 1, 2, 3, 4 or 5.
14. The *Escherichia Coli* of anyone of embodiment 4-13, wherein the subject is a vertebrate animal.
15. The *Escherichia Coli* of embodiment 14, wherein the vertebrate animal is selected from the group comprising humans, bovines, canines, felines, caprines, ovines, porcines, camelids, equines and avians.
16. The *Escherichia Coli* of embodiment 15, wherein the avian subject is any commercially or domestically raised avian.
17. The *Escherichia Coli* of embodiment 16, wherein the avian is selected from the group comprising chickens (including bantams), turkeys, ducks, geese, pheasants, quails, partridges, pigeons, guinea-fowls, ostriches, emus or pea-fowl.
18. An immunogenic composition comprising the *Escherichia Coli* of any preceding embodiment.
19. A vaccine composition comprising an immunogenically effective amount of at least one *Escherichia Coli* of any one of embodiments 1 to 17 and a pharmacologically acceptable carrier.
20. The vaccine composition or immunogenic composition of embodiments 18 or 19 wherein the vaccine compositions or immunogenic compositions are lyophilized or freeze-dried.
21. The vaccine composition or immunogenic composition of any one of embodiments 18 to 20, further comprising at least one adjuvant.
22. The vaccine composition or immunogenic composition of embodiment 21 wherein the adjuvant is selected from the group comprising Freund's complete adjuvant, Freund's incomplete adjuvant, vitamin E, non-ionic block polymers, muramyldipeptides, saponins, mineral oil, vegetable oil, carbopol aluminium hydroxide, aluminium phosphate, aluminium oxide, oil-emulsions saponins, vitamin- E solubilisate or any combination thereof.
23. The vaccine composition or immunogenic composition of embodiment 21, wherein the adjuvant is *E. coli* heat-labile toxin or Cholera toxin.
24. The vaccine composition or immunogenic composition of any one of embodiments 18 to 23, wherein the vaccine composition or immunogenic composition is administered intranasally, opthalmically, intradermally, intraperitoneally, intravenously, subcutaneously, orally, cloacally, by aerosol (spray vaccination) or intramuscularly.
25. The vaccine composition or immunogenic composition of any one of embodiments 18 to 24, wherein the vaccine composition or immunogenic comprises at least about 10³ to about 10⁵ attenuated bacteria, or about 10⁵ to about 10⁷ attenuated bacteria, or 10⁵ to about 10¹¹ attenuated bacteria or about 10⁷ to about 10⁹ attenuated bacteria, or about 10⁹ to about 10¹¹ attenuated bacteria, or about 10¹¹ to about 10¹³ attenuated bacteria, or about 10¹³ to about 10¹⁵ attenuated bacteria, or about 10¹⁵ to about 10¹⁷ attenuated bacteria, or about 10¹⁷ to about 10¹⁹, or at least about 10¹⁹ attenuated bacteria per dose.
26. A method for attenuating *Escherichia Coli,* the method comprising mutating at least one alr, asd, metC and dadX and murl gene and wherein the *E. coli* of any one of embodiments 1 to 17 persists in a subject.
27. A method of prevention or amelioration of a disease in a subject, the method comprising administering a therapeutically effective dose of a vaccine composition or immunogenic composition according to embodiments 18 to 25 to the subject.
28. A method of prophylaxis of a disease, the method comprising administering a therapeutically effective dose of a vaccine composition or immunogenic composition according to embodiments 18 to 25 to a subject in need of prophylaxis.
29. The use of a vaccine composition for the treatment or prophylaxis of disease wherein the vaccine composition comprises at least one *Escherichia Coli* of any one of embodiments 1 to 17.

## Claims

1. An *Escherichia Coli* attenuated by a mutation in any one of the following genes alr, asd, metC, dadX and murl, wherein the mutation renders the corresponding alr, asd, metC, dadX and murl protein nonfunctional.

2. An *Escherichia Coli* of claim 1, wherein the *Escherichia Coli* is a mutant live cell wall auxotrophic bacterial strain.

3. An *Escherichia Coli* of claim 1, wherein the mutation is a mutation in asd gene, or a mutation in alr, metC and dadX gene or a mutation in the murl gene, or a mutation in airs, asd, metC and dadX gene, or a mutation in alr, asd, metC, dadX and murl gene.

4. An *Escherichia Coli of claim 1*, wherein the attenuated *Escherichia Coli* can persist in a subject.

5. The *Escherichia Coli* of claim 1, wherein *Escherichia Coli* is extraintestinal *E*. *Coli,* preferably is Avian Pathogenic *Escherichia Coli* (APEC).

6. The *Escherichia Coli* of any preceding claim, wherein the air gene encodes a alanine racemase protein, or wherein the asd gene encodes a aspartate semialdehyde dehydrogenase protein or wherein the metC gene encodes a cystathionine beta- lyase protein or wherein the dadX gene encodes a alanine racemase protein or wherein the murl gene encodes a glutamate racemase protein.

7. The *Escherichia Coli* according to claims 5-6, wherein the APEC is APEC strain SOP017, deposited under the code DSM 35079 or a derivative thereof.

8. The *Escherichia Coli* of any preceding claim, comprising sequence ID NO: 1, 2, 3, 4 or 5.

9. An immunogenic composition comprising the *Escherichia Coli* of any preceding claim.

10. A vaccine composition comprising an immunogenically effective amount of at least one *Escherichia Coli* of any one of claims 1 to 8 and a pharmacologically acceptable carrier.

11. The vaccine composition or immunogenic composition of any one of claims 18 to 24, wherein the vaccine composition or immunogenic comprises at least about 10³ to about 10⁵ attenuated bacteria, or about 10⁵ to about 10⁷ attenuated bacteria, or 10⁵ to about 10¹¹ attenuated bacteria or about 10⁷ to about 10⁹ attenuated bacteria, or about 10⁹ to about 10¹¹ attenuated bacteria, or about 10¹¹ to about 10¹³ attenuated bacteria, or about 10¹³ to about 10¹⁵ attenuated bacteria, or about 10¹⁵ to about 10¹⁷ attenuated bacteria, or about 10¹⁷ to about 10¹⁹, or at least about 10¹⁹ attenuated bacteria per dose.

12. A method for attenuating *Escherichia Coli,* the method comprising mutating at least one alr, asd, metC and dadX and murl gene and wherein the *E. coli* of any one of claims 1 to 8 persists in a subject.

13. A vaccine composition or immunogenic for use in a method of prevention or amelioration of a disease in a subject, the method comprising administering a therapeutically effective dose of a vaccine composition or immunogenic composition according to claims 9 or 10 to the subject.

14. A method of prophylaxis of a disease, the method comprising administering a therapeutically effective dose of a vaccine composition or immunogenic composition according to claims 9 or 10 to a subject in need of prophylaxis.

15. The use of a vaccine composition for the treatment or prophylaxis of disease wherein the vaccine composition comprises at least one *Escherichia Coli* of any one of claims 1 to 8.
